(19) Europäisches Patentamt
European Patent Office
Office européen des brevets

(11) Veröffentlichungsnummer: **0 165 474**
**B1**

(12) # EUROPÄISCHE PATENTSCHRIFT

(45) Veröffentlichungstag der Patentschrift: **02.08.89**

(51) Int. Cl.⁴: **A 61 K 31/495** // C07D215/56

(21) Anmeldenummer: **85106051.7**

(22) Anmeldetag: **17.05.85**

(54) **Immunstimulierende Mittel.**

(30) Priorität: **30.05.84 DE 3420116**

(43) Veröffentlichungstag der Anmeldung: **27.12.85 Patentblatt 85/52**

(45) Bekanntmachung des Hinweises auf die Patenterteilung: **02.08.89 Patentblatt 89/31**

(84) Benannte Vertragsstaaten: **BE CH DE FR GB IT LI**

(56) Entgegenhaltungen:
**EP-A-0 059 698**
**DE-A-2 806 879**
**FR-A-2 342 067**

**Die Akte enthält technische Angaben, die nach dem Eingang der Anmeldung eingereicht wurden und die nicht in dieser Patentschrift enthalten sind.**

(73) Patentinhaber: **BAYER AG, D-5090 Leverkusen 1 Bayerwerk (DE)**

(72) Erfinder: **Grohe, Klaus, Dr., Am Wasserturm 10, D-5068 Odenthal (DE)**
Erfinder: **Klimetzek, Volker, Dr., Kantstrasse 64, D-5620 Velbert 15 (DE)**
Erfinder: **Metzger, Karl Georg, Dr., Pahlkestrasse 75, D-5600 Wuppertal 1 (DE)**
Erfinder: **Stünkel, Klaus Georg, Dr., Am Eckbusch 55, D-5600 Wuppertal 1 (DE)**
Erfinder: **Zeiler, Hans- Joachim, Dr., Elsbeeker Strasse 46, D-5620 Velbert 15 (DE)**

EP 0 165 474 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1989

**Beschreibung**

Die Erfindung betrifft die Verwendung bekannter 1-Cyclopropylchinolon-3-carbonsäuren für die Herstellung immunstimulierender Mittel.

Es ist bereits bekannt geworden, daß bestimmte 1-Cyclopropylchinolon-3-carbonsäuren gute antibakterielle Wirkungen besitzen [DE-A-3 142 854].

Es wurde nun gefunden, daß die bekannten 1-Cyclopropylchinolon-3-carbonsäuren der allgemeinen Formel I und deren physiologisch verträglichen Säureadditionssalze und Alkalisalze immunstimulierende Wirkungen aufweisen. Überraschenderweise zeigen sie neben sehr guten antibakteriellen Wirkungen die genannten immunstimulierenden Wirkungen in höherem Maße als bekannte immunstimulierende Stoffe. Die erfindungsgemäßen Mittel stellen somit eine Bereicherung der Pharmazie dar.

Die erfindungsgemäßen Wirkstoffe der allgemeinen Formel sind bekannt [DE-A-3 142 854, DE-A-3 033 157] und sind Gegenstand eines eigenen älteren Vorschlages (Deutsche Patentanmeldung 3 318 145.4 vom 16.5.1983).

In der EP-A-0 059 698 Al wird die immunstimulierende Wirkung von heterocyclischen Carboxamiden beschrieben.

Gegenstand der Erfindung ist die Verwendung bekannter 1-Cyclopropylchinolon-3-carbonsäuren der allgemeinen Formel I

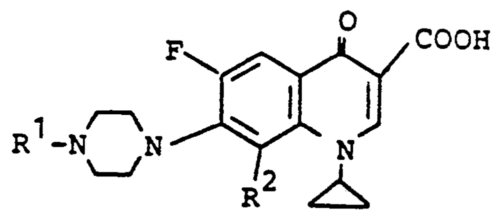

(I)

in der

$R^1$ für Wasserstoff, Methyl, Ethyl oder β-Hydroxyethyl

und

$R^2$ für Wasserstoff, Chlor oder Fluor steht,

und/oder deren physiologisch verträglichen Säureadditionssalze und Alkalisalze für die Herstellung eines immunstimulierenden Mittels.

Insbesondere seien als Wirkstoffe genannt:

1-Cyclopropyl-6-fluor-1,4-dihydro-4-oxo-7-(1-piperazinyl)-3-chinolincarbonsäure (Ciprofloxacin);

1-Cyclopropyl-6-fluor-1,4-dihydro-4-oxo-7-(4-ethyl-1-piperazinyl)-3-chinolincarbonsäure;

1-Cyclopropyl-6,8-difluor-1,4-dihydro-4-oxo-7-(1-piperazinyl)-4-chinolincarbonsäure;

1-Cyclopropyl-6,8-difluor-1,4-dihydro-4-oxo-7-(4-methyl-1-piperazinyl)-3-chinolincarbonsäure.

Folgende Patientengruppen können beispielsweise mit Erfolg mit den erfindungsgemäßen Mitteln behandelt werden:

Patienten, die an Infektions- oder Alters-bedingten, Tumor-induzierten oder durch Cytostatika hervorgerufenen Immunschwächen leiden.

Außerdem kommen postoperative und posttraumatische immunologische Abwehrschwächen vor. Die Behandlung von Patienten mit solchen erworbenen Immundefekten ist nicht befriedigend gelöst.

Alle diese Patienten benötigen Medikationen, die die körpereigene Abwehr unterstützen bzw. normalisieren.

Zur vorliegenden Erfindung gehören pharmazeutische Zubereitungen, die neben nicht toxischen, inerten pharmazeutisch geeigneten Trägerstoffen einen oder mehrere erfindungsgemäße Wirkstoffe enthalten oder die aus einem oder mehreren erfindungsgemäßen Wirkstoffen bestehen sowie Verfahren zur Herstellung dieser Zubereitungen.

Zur vorliegenden Erfindung gehören auch pharmazeutische Zubereitungen in Dosierungseinheiten. Dies bedeutet, daß die Zubereitungen in Form einzelner Teile, z. B. Tabletten, Dragees, Kapseln, Pillen, Suppositorien und Ampullen vorliegen, deren Wirkstoffgehalt einem Bruchteil oder einem Vielfachen einer Einzeldosis entspricht. Die Dosierungseinheiten können z. B. 1, 2, 3 oder 4 Einzeldosen oder 1/2, 1/3 oder 1/4 einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Unter nicht toxischen, inerten pharmazeutisch geeigneten Trägerstoffen sind feste, halbfeste oder flüssige Verdünnungsmittel, Füllstoffe und Formulierungshilfsmittel jeder Art zu verstehen.

Als bevorzugte pharmazeutische Zubereitungen seien Tabletten, Dragees, Kapseln, Pillen, Granulate, Suppositorien, Lösungen, Suspensionen und Emulsionen, Pasten, Salben, Gele, Cremes, Lotions, Puder und Sprays genannt.

Tabletten, Dragees, Kapseln, Pillen und Granulate können den oder die Wirkstoffe neben den üblichen Trägerstoffen enthalten, wie (a) Füll- und Streckmittel, z. B. Stärken, Milchzucker, Rohrzucker, Glukose, Mannit und Kieselsäure, (b) Bindemittel, z. B. Carboxymethylcellulose, Alginate, Gelatine, Polyvinylpyrrolidon, (c)

Feuchthaltemittel, z. B. Glycerin, (d) Sprengmittel, z. B. Agar-Agar, Calciumcarbonat und Natriumbicarbonat, (e) Lösungsverzögerer, z. B. Paraffin und (f) Resorptionsbeschleuniger, z. B. quaternäre Ammoniumverbindungen, (g) Netzmittel, z. B. Cetylalkohol,Glycerinmonostearat, (h) Adsorptionsmittel, z. B. Kaolin und Bentonit und (i) Gleitmittel, z. B. Talkum, Calcium- und Magnesiumstearat und feste Polyethylenglykole oder Gemische der unter (a) bis (i) aufgeführten Stoffe.

Den Tabletten, Dragees, Kapseln, Pillen und Granulate können mit den üblichen gegebenenfalls Opakisierungsmittel enthaltenden Überzügen und Hüllen versehen sein und auch so zusammengesetzt sein, daß sie den oder die Wirkstoffe nur oder bevorzugt in einem bestimmten Teil des Intestinaltraktes, gegebenenfalls verzögert abgeben, wobei als Einbettungsmassen, z. B. Polymersubstanzen und Wachse verwendet werden können.

Der oder die Wirkstoffe können gegebenenfalls mit einem oder mehreren der oben angegebenen Trägerstoffen auch in mikroverkapselter Form vorliegen.

Suppositorien können neben dem oder den Wirkstoffen die üblichen wasserlöslichen oder wasserunlöslichen Trägerstoffe enthalten, z. B. Polyethylenglykole, Fette, z. B. Kakaofett und höhere Ester (z. B. $C_{14}$-Alkohol mit $C_{16}$-Fettsäure) oder Gemische dieser Stoffe.

Salben, Pasten, Cremes und Gele können neben dem oder den Wirkstoffen die üblichen Trägerstoffe enthalten, z. B. tierische und pflanzliche Fette, Wachse, Paraffine, Stärke, Tragant, Cellulosederivate, Polyethylenglykole, Silicone, Bentonite, Kieselsäure, Talkum und Zinkoxid oder Gemische dieser Stoffe.

Puder und Sprays können neben dem oder den Wirkstoffen die üblichen Trägerstoffe enthalten, z. B. Milchzucker, Talkum, Kieselsäure, Aluminiumhydroxid, Calciumsilikat und Polyamidpulver oder Gemische dieser Stoffe. Sprays können zusätzlich die üblichen Treibmittel z. B. Chlorfluorkohlenwasserstoffe enthalten.

Lösungen und Emulsionen können neben dem oder den Wirkstoffen die üblichen Trägerstoffe wie Lösungsmittel, Lösungsvermittler und Emulgatoren, z. B. Wasser, Ethylalkohol, Isopropylalkohol, Ethylcarbonat, Ethylacetat, Benzylalkohol, Benzylbenzoat, Propylenglykol, 1,3-Butylenglykol, Dimethylformamid, Öle, insbesondere Baumwollsaatöl, Erdnußöl, Maiskeimöl, Olivenöl, Ricinusöl und Sesamöl, Glycerin, Glycerinformal, Tetrahydrofurfurylalkohol, Polyethylenglykole und Fettsäureester des Sorbitans oder Gemische dieser Stoffe enthalten.

Zur parenteralen Applikation können die Lösungen und Emulsionen auch in steriler und blutisotonischer Form vorliegen.

Suspensionen können neben dem oder den Wirkstoffen die üblichen Trägerstoffe wie flüssige Verdünnungsmittel, z. B. Wasser, Ethylalkohol, Propylenglykol, Suspendiermittel, z. B. ethoxylierte Isostearylalkohole, Polyoxyethylensorbit- und Sorbitanester, mikrokristalline Cellulose, Aluminiummetahydroxid, Bentonit, Agar-Agar und Tragant oder Gemische dieser Stoffe enthalten.

Die genannten Formulierungsformen können auch Färbemittel, Konservierungsstoffe sowie geruchs- und geschmacksverbessernde Zusätze, z. B. Pfefferminzöl und Eukalyptusöl und Süßmittel, z. B. Saccharin enthalten.

Die therapeutisch wirksamen Verbindungen sollen in den oben aufgeführten pharmazeutischen Zubereitungen vorzugsweise in einer Konzentration von etwa 0,1 bis 99,5, vorzugsweise von 0,5 bis 95 Gew.-% der Gesamtmischung vorhanden sein.

Die oben aufgeführten pharmazeutischen Zubereitungen können außer den erfindungsgemäßen Wirkstoffen auch weitere pharmazeutische Wirkstoffe enthalten.

Die Herstellung der oben aufgeführten pharmazeutischen Zubereitungen erfolgt in üblicher Weise nach bekannten Methoden, z. B. durch Mischen des oder der Wirkstoffe mit dem oder den Trägerstoffen.

Zur vorliegenden Erfindung gehört auch die Verwendung der erfindungsgemäßen Wirkstoffe sowie von pharmazeutischen Zubereitungen, die einen oder mehrere erfindungsgemäße Wirkstoffe enthalten, in der Human- und Veterinärmedizin zur Verhütung, Besserung und/oder Heilung der oben angeführten Erkrankungen.

Die Wirkstoffe oder die pharmazeutischen Zubereitungen können lokal, oral, parenteral, intraperitoneal und/oder rectal, vorzugsweise parenteral, insbesondere intravenös appliziert werden.

Im allgemeinen hat es sich sowohl in der Human- als auch in der Veterinärmedizin als vorteilhaft erwiesen, den oder die erfindungsgemäßen Wirkstoffe in Gesamtmengen von etwa 10 bis etwa 300, vorzugsweise 2,5 bis 25 mg/kg Körpergewicht je 24 Stunden, gegebenenfalls in Form mehrerer Einzelgaben zur Erzielung der gewünschten Ergebnisse zu verabreichen.

Es kann jedoch erforderlich sein, von den genannten Dosierungen abzuweichen und zwar in Abhängigkeit von der Art und dem Körpergewicht des zu behandelnden Objekts, der Art und der Schwere der Erkrankung, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der obengenannten Menge Wirkstoff auszukommen, während in anderen Fällen die oben angeführte Wirkstoffmenge überschritten werden muß.

Die Festlegung der jeweils erforderlichen optimalen Dosierung und Applikationsart der Wirkstoffe kann durch jeden Fachmann aufgrund seines Fachwissens leicht erfolgen.

Die erfindungsgemäß verwendeten Wirkstoffe weisen eine ausgeprägte Abwehr-steigernde Wirkung auf. Sie steigern antigenspezifisch die humorale, Antikörper-vermittelte und die Zell-vermittelte Abwehr des Immunsystems.

Diese Ergebnisse wurden anhand der nachfolgenden Versuchsordnung erhalten:

1. Steigerung der primären humoralen Immunität in vivo gegen das lösliche Antigen Ovalbumin am Beispiel Ciprofloxacin.

NMRI-Mäuse wurden mit einer suboptimalen Antigendosis (1 µg/Tier, Tag 0) subcutan (s.c.) immunisiert. Bei suboptimaler Antigenstimulation wird nur eine geringe Zahl von Lymphozyten der Tiere zur Antikörpersynthese angeregt. Die zusätzliche Behandlung der Tiere mit Ciprofloxacin ist in der Lage, bei einer einmaligen Applikation von 10 - 80 mg/kg subcutan den Antikörpertiter im Serum der Tiere leicht, aber signifikant zu steigern (Tabelle 1). Die Antikörperbestimmung erfolgte durch indirekte Hämagglutination am Tag 10. Der Effekt der Behandlung wird durch den geometrischen Mittelwert der $log_2$-Titer ausgedrückt.

Der immunstimulierende Effekt von Ciprofloxacin ist im Gegensatz zu anderen, z. B. bakteriellen Immunstimulantien wie LPS aus Gram-negativen Bakterien, Antigen-abhängig, d. h. die Substanz bewirkt nur in Verbindung mit einem antigenen Reiz (hier Ovalbumin) die Induktion der Antikörpersynthese.

2. Steigerung der unspezifischen, Mitogen-induzierten Stimulation von Maus-Milzlymphozyten (MML).

Balb/c-Mäuse wurden für die Dauer von 7 Tagen mit verschiedenen Dosen Ciprofloxacin s.c. vorbehandelt. ML derart vorbehandelter Tiere wurden am 8. Tag ex vivo mit den T-Zellmitogenen ConA (5 µg/ml) und PHA (10 µg/ml) stimuliert. Nach 48 Stunden wurden die Kulturen für weitere 16 Stunden mit $^3$H-Thymidin markiert und geerntet. Die Inkorporation des radioaktiven Thymidins in die neu synthetisierte DNA gilt als Maß für die erfolgte Lymphozytenproliferation. Die Ergebnisse zeigen eine deutliche Proliferationszunahme innerhalb des Dosisbereichs von 1 - 80 mg/kg (Tabelle 2).

Tabelle 1: Einfluß von Ciprofloxacin auf die Antikörpersynthese gegen das lösliche Antigen Ovalbumin

| Wirkstoff | Dosis (mg/kg) Ø | 1 | 5 | 10 | 20 | 80 |
|---|---|---|---|---|---|---|
| | Hämaggl. Titer $log_2$) | | | | | |
| Ciprofloxacin | 4,6 | 4,8 | 4,8 | 5,4[1)] | 5,4[1)] | 5,4[1)] |

[1)] statistisch signifikant $p < 0,05$

**Tabelle 2:** Unspezifische, mitogene Stimulation von Maus-Milzlymphozyten Ciprofloxacin-vorbehandelter Mäuse

| Dosis (mg/kg) | Experiment 1 ConA | PHA | Ø | Experiment 2 ConA | PHA | Ø |
|---|---|---|---|---|---|---|
| | $^3$H-Thymidin-Einbau (CPM) | | | | | |
| Ø | 49.208 | 39.620 | 455 | 57.148 | 32.372 | 1.228 |
| 1 | 122.421 | 37.093 | 424 | 84.374 | 29.056 | 2.534 |
| 5 | 79.249 | 75.886 | 320 | 84.368 | 45.400 | 640 |
| 20 | 57.009 | 46.289 | 342 | 68.826 | 34.827 | 416 |
| 80 | 60.963 | 42.286 | 620 | 61.780 | 22.818 | 700 |

Ein ähnliches Proliferationsverhalten wurde auch bei Behandlung in vitro der MML-Mitogen-Kulturen nicht vorbehandelter Mäuse erzielt.

3. Steigerung der spezifischen, T-Zell-vermittelten, allogenen Immunantwort (Mixed Lymphocyte Culture-MLC) von MML

C 57 bl-Mäuse wurden für die Dauer von 7 Tagen mit verschiedenen Dosen Ciprofloxacin s.c. vorbehandelt. Milzen derart vorbehandelter Tiere wurden am 8. Tag nach Entnahme suspendiert und T-Lymphozyten aus diesen Suspensionen jeweils durch eine Nylon-Wool-Säulenpassage angereichert [modifiziert nach S. A. Eisen, H. J. Wedner und C. W. Parker, Immunological Communications 1, 571, (1972].

Diese T-Zellfraktionen wurden als Responder in der Einweg-MLC eingesetzt. Als Stimulatorzellen wurden Mitomycin C-behandelte MM-Zellen von DBA-Mäusen eingesetzt. Die Kulturen wurden nach 72 Stunden mit $^3$H-Thymidin versetzt und nach weiteren 16 Stunden geerntet [modifiziert nach D. B. Amos und F. K. Bach, J. Exp. Med. 128, 623 (1988)]. Die $^3$H-Thymidin-Inkorporationsmessungen ergaben auch in diesem Testsystem eine signifikante Proliferationszunahme gegenüber der Kontrolle in dem Dosierbereich von 1 - 80 mg/kg.

Behandlungen in vitro von derartigen Kulturen nicht vorbehandelter Tiere haben gleichermaßen eine gesteigerte T-Lymphozytenproliferation im Dosisbereich von 0,01 - 10 µg Substanz/ml zur Folge.

4. Mitogene und allogene T-Lymphozytenaktivierung humaner Blutlymphozyten (PBL).

Lymphozyten wurden dazu aus dem heparinisierten Blut gesunder Spender durch Dichtegradientenzentrifugation isoliert. Die so erhaltenen Zellen wurden in Parallelsätzen mit den Mitogenen PHA (5 µg/m1) und ConA (5 µg/m1) ± der ausgewählten Substanz kultiviert, nach 72 Stunden $^3$H-thymidiert und nach weiteren 16 Stunden geerntet. Mitogen-Responsezunahmen wurden in dem Dosisbereich von 1 - 50 µg Substanz/ml beobachtet (Tabelle 3).

Isolierte, unfraktionierte PBL, die simultan mit der exemplarisch ausgewählten Substanz der oben beschriebenen Derivate bei verschiedenen Dosen in der Einweg-MCL aktiviert wurden, zeigen nach 136 Stunden Kulturdauer gegenüber den Kontrollen Proliferationssteigerungen in dem Dosisbereich von 0,1 - 30 µg Substanz/ml (Tabelle 4).

Im Gegensatz zu anderen, konventionellen Immunstimulantien, wie z. B. das oben erwähnte LPS, haben diese Verbindungen keine mitogenen Eigenschaften, d. h. sie wirken nur in Verbindung mit einem zusätzlich induzierten Reiz.

**Tabelle 3**: Einfluß von Ciprofloxacin auf die unspezifische, mitogene Aktivierung humaner Blutlymphozyten

| | Experiment 1 [1] | | | Experiment 2 [1] | | | Experiment 3 [2] | | |
|---|---|---|---|---|---|---|---|---|---|
| Dosis (µg/ml) | PHA | ConA | Ø | PHA | ConA | Ø | PHA | ConA | Ø |
| | | | | $^3$H-Thymidin-Einbau (CPM) | | | | | |
| Ø | 102.566 | 41.592 | 330 | 133.182 | 57.604 | 693 | 44.028 | 17.866 | 1081 |
| 1 | 109.750 | 46.576 | 335 | 125.109 | 56.166 | 479 | 111.379 | 31.276 | 844 |
| 3 | 105.730 | 45.919 | 386 | 112.918 | 65.598 | 447 | 122.275 | 34.096 | 884 |
| 10 | 144.867 | 52.455 | 333 | 143.780 | 73.484 | 460 | 145.795 | 36.080 | 1061 |
| 30[3] | 171.005 | 36.546 | 437 | 176.089 | 56.454 | 344 | 129.103 | 13.790 | 463 |

[1] mit Lymphozyten aus Frischblut
[2] mit aufgetauten, stickstoffgelagerten Lymphozyten
[3] bereits cytotoxische Effekte vorhanden

**Tabelle 4**: Einfluß von Ciprofloxacin auf die spezifische, allogene Aktivierung humaner Blutlymphozyten

| Versuchsansätze | Dosis (µg/ml) | | | | | | |
|---|---|---|---|---|---|---|---|
| | 0 | 0,1 | 0,3 | 1 | 3 | 10 | 30[2] |
| | $^3$H-Thymidineinbau (CPM) | | | | | | |
| Nr. 1 KO[1] | 4.890 | | | | | | |
| Test | 17.252 | 21.310 | 26.964 | 28.982 | 27.841 | 32.733 | 28.900 |
| Nr. 2 KO | 946 | | | | | | |
| Test | 42.292 | 64.461 | 82.315 | 87.12 | 82.933 | 72.662 | 34.988 |
| Nr. 3 KO | 245 | | | | | | |
| Test | 11.679 | 21.768 | 26.564 | 20.314 | 13.964 | 15.142 | 4.632 |

[1] autologe Kontrolle: Mit Mitomycin C behandelten Lymphozyten als Stimulator
[2] bereits zytotoxische Effekte vorhanden

## Herstellungsbeispiele

### Beispiel 1

**Ciprofloxacin**

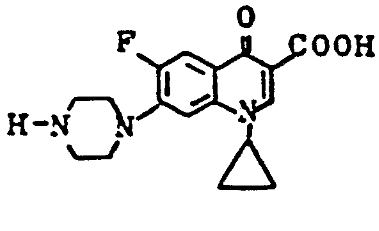

Ein Gemisch von 19,7 g 7-Chlor-1-cyclopropyl-6-fluor-1,4-dihydro-4-oxo-chinolin-3-carbonsäure, 30,1 g wasserfreiem Piperazin und 100 ml Dimethylsulfoxid wird 2 h auf 135 - 140°C erhitzt. Das Lösungsmittel wird i. Feinvakuum abdestilliert, der Rückstand in $H_2O$ suspendiert, abgesaugt und mit Wasser gewaschen. Zur weiteren Reinigung wird das feuchte Rohprodukt mit 100 ml Wasser aufgekocht, bei Raumtemperatur abgesaugt, mit $H_2O$ gewaschen und im Vakuumtrockenschrank über $CaCl_2$ bei 100°C bis zur Gewichtskonstanz

getrocknet. Man erhält 19,6 g 1-Cyclopropyl-6-fluor-1,4-dihydro-4-oxo-7-(1-piperazinyl)-chinolin-3-carbonsäure vom Zersp. 255 - 257° C.

Die nach Beispiel 1 hergestellte Verbindung wird in 50 ml 10-proz. Salzsäure heiß gelöst. Zur filtrierten Lösung gibt man 150 ml Ethanol, kühlt mit Eis, saugt ab und wäscht mit Alkohol und trocknet i. Vakuum bei 100° C.

Es werden 18,5 g 1-Cyclopropyl-6-fluor-1,4-dihydro-4-oxo-7-(1-piperazinyl)-chinolin-3-carbonsäurehydrochlorid als farblose Kristalle vom Zersp. 308 - 310° C erhalten.

**Beispiel 2**

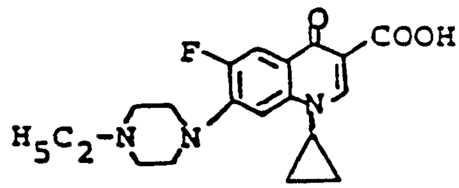

x HI

Ein Gemisch von 1,2 g 1-Cyclopropyl-6-fluor-1,4-dihydro-4-oxo-7-(1-piperazinyl)-chinolin-3-carbonsäure, 1,13 g Ethyliodid, 0,73 g Triethylamin und 20 ml N,N-Dimethylformamid wird 2,5 h auf 70 - 80° C erhitzt. Das Lösungsmittel wird i. Vakuum abdestilliert und der Rückstand in Wasser suspendiert. Man saugt ab, wäscht mit $H_2O$ nach und drückt auf Ton an. Es werden 1,15 g 1-Cyclopropyl-6-fluor-1,4-dihydro-4-oxo-7-(4-ethyl-1-piperazinyl)-chinolin-3-carbonsäure-hydroiodid vom Zersp. 306° C erhalten.

**Beispiel 3**

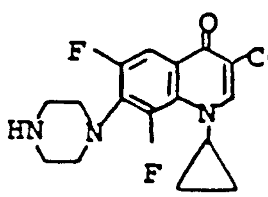

x HCl

Ein Gemisch aud 2,83 g (0,01 Mol) 1-Cyclopropyl-6,7,8-trifluor-1,4-dihydro-4-oxo-3-chinolincarbonsäure, 4,4 g (0,051 Mol) wasserfreies Piperazin und 30 ml trockenem Pyridin wird 6 Stunden refluxiert. Das Lösungsmittel wird im Vakuum abgezogen, der Rückstand in 25 ml Wasser aufgenommen, unter Eiskühlung mit konzentrierter Salzsäure auf pH 1 gestellt, der Niederschlag kalt abgesaugt, mit kalter 10 %-iger Salzsäure und Ethanol gewaschen. Nach dem Trocknen im Vakuum bei 100° C erhält man 3,05 g 1-Cyclopropyl-6,8-difluor-1,4-dihydro-4-oxo-7-(1-piperazinyl)-3-chinolincarbonsäure-hydrochlorid vom Zersetzungspunkt 354 - 355° C.

**Beispiel 4**

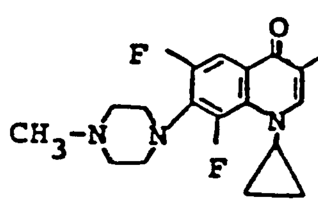

x HCl

Man setzt 2,83 g (0,01 Mol) 1-Cyclopropyl-6,7,8-trifluor-1,4-dihydro-4-oxo-3-chinolincarbonsäure mit 4 g (0,04 Mol) N-Methylpiperazin analog Beispiel 3 um und isoliert 3,6 g 1-Cyclopropyl-6,8-difluor-1,4-dihydro-4-oxo-7-(4-methyl-1-piperazinyl)-3-chinolincarbonsäure-hydrochlorid vom Zersetzungspunkt 300 - 303° C.

## Patentansprüche

1. Verwendung von 1-Cyclopropylchinolon-3-carbonsäuren der allgemeinen Formel

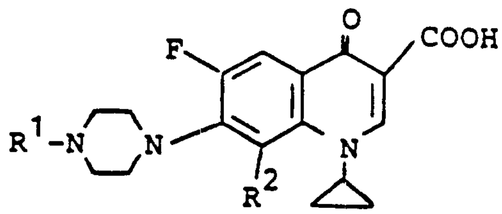

(I)

in der

$R^1$ für Wasserstoff, Methyl, Ethyl oder β-Hydroxyethyl und
$R^2$ für Wasserstoff, Chlor oder Fluor steht,
und/oder deren physiologiscch verträglichen Säureadditionssalzen und Alkalisalzen für die Herstellung eines immunstimulierenden Mittels.

2. Verwendung von 1-Cyclopropyl-6-fluor-1,4-dihydro-4-oxo-7-(1-piperazinyl)-3-chinolincarbonsäure und /oder deren physiologisch verträglichen Säureadditionssalzen und Alkalisalzen für die Herstellung eines immunstimulierenden Mittels.

## Claims

1. Use of 1-cyclopropylquinolone-3-carboxylic acids of the general formula I

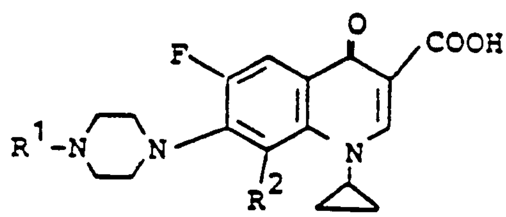

(I)

in which

$R^1$ represents hydrogen, methyl, ethyl or β-hydroxyethyl, and
$R^2$ represents hydrogen, chlorine or fluorine,
and/or their physiologically tolerated acid addition salts and alkali metal salts for the preparation of an immunostimulating agent.

2. Use of 1-cyclopropyl-6-fluoro-1,4-dihydro-4-oxo-7-(1-piperazinyl)-3-quinolinecarboxylic acid and/or its physiologically tolerated acid addition salts and alkali metal salts for the preparation of an immunostimulating agent.

## Revendications

1. Utilisation des acides 1-cyclopropylquinolone-3-carboxyliques de formule générale I

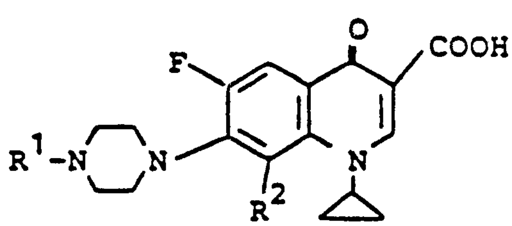

(I)

dans laquelle

$R^1$ représente l'hydrogène, un groupe méthyle, éthyle ou bêtahydroxyéthyle, et
$R^2$ représente l'hydrogène, le chlore ou le fluor,

et/ou de leurs sels formés par addition avec des acides et sels alcalins acceptables pour l'usage pharmaceutique, pour la préparation d'un agent immunostimulant.

2. Utilisation de l'acide 1-cyclopropyl-6-fluoro-1, 4-dihydro-4-oxo-7-(1-pipérazinyl) -3-quinoléine-carboxylique et/ou de ses sels formés par addition avec des acides et sels alcalins acceptables pour l'usage pharmaceutique, pour la préparation d'un agent immunostimulant.